# EUROPEAN PATENT SPECIFICATION

(11) **EP 1 601 407 B1**
(45) Date of publication and mention of the grant of the patent: **09.11.2011**
(21) Application number: 04701245.5
(22) Date of filing: 09.01.2004
(51) Int. Cl.: A61M 25/06

(54) **HOLLOW STYLET**
HOHLER MANDRIN
STYLET CREUX

(30) Priority: 13.01.2003 US 439728 P
(43) Date of publication of application: 07.12.2005
(73) Proprietor: BrainLAB AG, 85622 Feldkirchen (DE)
(72) Inventor: BRADY, Martin, Phoenix, MD 21131 (US); PEDAIN, Christoph, 81667 München (DE)
(74) Representative: Rögner, Jürgen
(86) International application number: PCT/US2004/000589
(87) International publication number: WO 2004/062718

(56) References cited:
- US-A- 4 046 144
- US-A- 5 098 411
- US-A- 5 690 117

## Description

### TECHNICAL FIELD

This document relates generally to catheters, and particularly, but not by way of limitation, to a device for use in inserting a catheter into tissue.

### BACKGROUND

Catheters are thin tubes that can be inserted into a patient's body, such as to deliver therapeutic agent(s), diagnostic sensor(s), etc. to remote locations inside the body. Infusion catheters are sometimes used for infusing a therapeutic agent into tissue, such as brain tissue. Such infusion delivers the agent through the catheter directly to a specific target site within the tissue. Infusion is particularly important for delivering a substance to the brain, since a blood-brain barrier limits the effectiveness of systemic therapy by preventing large molecules from passing from the bloodstream into the brain. The infusion catheter may remain in place in the tissue for hours or days.

One typical method - not forming part of the invention - for placing a catheter into brain tissue for direct infusion is described below. The catheter is inserted through an access opening in the skull, and advanced from this access point in a straight trajectory, directly through the tissue, until a distal end of the catheter reaches a target location. Then, to prevent infection at the access site, a portion of the catheter remaining outside the skull is tunneled beneath the scalp to a position several centimeters away from the access site, and the skin over the access site is closed. The agent can then be introduced through the catheter tube by connecting a filled syringe or pump to a proximal end of the catheter.

US 5 690 117 shows a system according to the preamble of claim 1.

Catheters used for direct infusion are often made of flexible polymer materials. This allows them to undergo sharp bends for tunneling. It also ensures that a movement of the proximal end of the tube, outside the skull, will not transfer any significant force to the distal end resting in position in the tissue at the target site. One disadvantage of such flexible material is that it is too flaccid to be pushed in a straight trajectory through the tissue to the target position.

To overcome this problem, such catheters are generally inserted using a guide wire, which is sometimes called a stylet or a trocar. Stylets are straight, rigid wires of diameter similar to or slightly less than the interior diameter of the catheters for which they are designed. They are made out of comparatively stiff materials, e.g., stainless steel or other metal(s). A typical stylet diameter is around one millimeter. The stylet is inserted into the catheter to stiffen it while it is being introduced into tissue and advanced to the target location.

After the catheter is introduced into tissue and reaches the target location, the stylet is removed from the catheter. This leaves only the catheter in place in the patient's tissue. The present inventors have recognized that one resulting problem is that removing the stylet leaves behind air filling the interior volume of the catheter. However, such air cannot easily be removed. Any fluid later introduced into the catheter for infusion will first push this remaining air into the tissue. Air infused into the tissue can adversely effect the infusion of a fluid agent. For example, bubbles of air in the tissue can cause the infusing fluid to distribute in an unpredictable and/or undesirable pattern. For these and other reasons, which will become apparent upon reading the following detailed description and viewing the drawings that form a part thereof, the present inventors have recognized an unmet need for improved catheters, catheter insertion devices, and ancillary tools and methods.

### SUMMARY

This document discusses, among other things, improved catheters, catheter insertion devices, and ancillary tools and methods -the latter not forming part of the invention - for reducing or eliminating the amount of air that is introduced into the catheter interior during withdrawal of the stylet. In one example, a hollow but rigid tube, which may be referred to as a "hollow stylet", replaces the solid, rigid guide wire. In one example, the hollow stylet has an outer diameter that is similar to an inner diameter of the flaccid hollow tubular catheter, such that no appreciable amount of air lies between the outer wall of the hollow stylet and the inner wall of the flaccid catheter. Thus, the hollow stylet can be conceptualized as completely filling the inside of the catheter tube, with a fluid completely filling the inside of the hollow stylet. In one example, a distal end of the hollow stylet is open. However, by temporarily closing a proximal end of the fluid-filled hollow stylet, fluid is discouraged from leaking out the open distal end port until the proximal end of the hollow stylet is opened, such as before it is extracted from the infusion catheter. One possible realization of the hollow stylet includes a needle cannula with an unsharpened blunt distal end hole. In this example, the needle cannula is designed such that its outer diameter fits somewhat snugly within the inner diameter of the flaccid infusion catheter. In this example, the needle cannula is configured with a length that is equal to or greater than that of the flaccid infusion catheter.

Before inserting the infusion catheter, the hollow stylet is inserted therewithin. The interior of the hollow stylet is filled with the fluid therapeutic agent, or with some other fluid, such as sterile saline. A proximal region of the hollow stylet is then sealed or otherwise closed to retain the fluid therewithin. During insertion of the infusion catheter, the hollow stylet performs the same catheter-stiffening function as a normal solid stylet. After insertion, the proximal end of the hollow stylet is unsealed or opened. Then the hollow stylet is removed from the infusion catheter. This creates a resulting vacuum at the distal end of the infusion catheter as the hollow stylet is extracted from the infusion catheter. This resulting vacuum draws the fluid out of the interior of the hollow stylet. The drawn-out fluid fills the infusion catheter interior. This reduces or eliminates the problem of air being left behind by removal of a solid stylet.

In one example, there is a seal between the interior wall of the infusion catheter and the exterior wall of the hollow stylet. This seal prevents air from entering the infusion catheter from its proximal end. In one example, the proximal end of the hollow stylet is connected to a fluid-filled tube or other reservoir. This ensures that the hollow stylet introduces enough fluid with which to fill the entire interior volume of the infusion catheter during removal of the hollow stylet. The proximal end of the infusion catheter is then connected to the infusion source carrying a therapeutic fluid agent.

### BRIEF DESCRIPTION OF THE DRAWINGS

In the drawings, which are not necessarily drawn to scale, like numerals describe substantially similar components throughout the several views. Like numerals having different letter suffixes represent different instances of substantially similar components. The drawings illustrate generally, by way of example, but not by way of limitation, various embodiments discussed in the present document.
Figure 1 is a schematic diagram illustrating generally a partial cross-section of a portion of a system including a flexible catheter and a hollow stylet.
Figure 2 is a flow chart illustrating generally one method of using a system that includes a flexible catheter and a hollow stylet.
Figure 3 is a schematic diagram illustrating generally a portion of the flexible catheter and a portion of the hollow stylet, in which a proximal end of the catheter seals snugly around the hollow stylet, but more distal portions of the catheter are not as snugly fitted around the hollow stylet.

### DETAILED DESCRIPTION

In the following detailed description, reference is made to the accompanying drawings which form a part hereof, and in which is shown by way of illustration specific embodiments in which the invention may be practiced. These embodiments are described in sufficient detail to enable those skilled in the art to practice the invention, and it is to be understood that the embodiments may be combined, or that other embodiments may be utilized and that structural, logical and electrical changes may be made without departing from the scope of the present invention. The following detailed description is, therefore, not to be taken in a limiting sense, and the scope of the present invention is defined by the appended claims.

In this document, the terms "a" or "an" are used, as is common in patent documents, to include one or more than one.

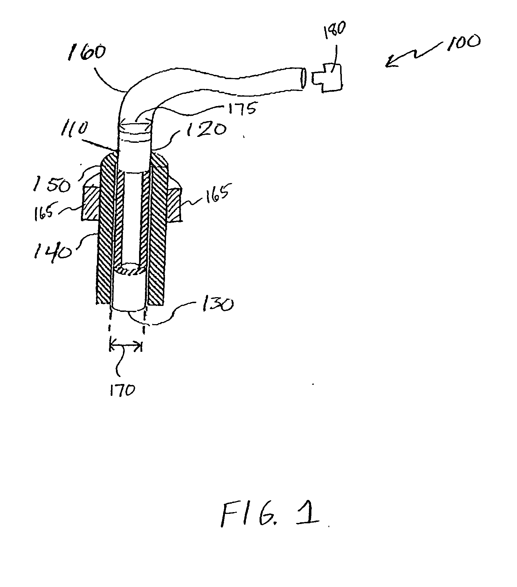

Figure **1** is a schematic diagram illustrating generally, by way of example, but not by way of limitation, a system **100** including an elongate flexible tubular infusion or other catheter **140** and an elongate hollow stylet tube **110.** In this example, the hollow tube stylet **110** is inserted into the catheter **140** for use as a stiffening guidewire to guide the catheter **140** to a target within a patient's brain or other tissue. In one example, a proximal end **120** of the hollow-tube stylet **110** is connected to a separate or integral fluid reservoir **160,** which may be enclosed (e.g., a bladder) or open. This ensures that enough fluid can be provided during removal of the hollow-tube stylet **110** from the infusion catheter **140** to fill the interior of the infusion catheter. This, in turn, prevents air from entering the infusion catheter and subsequently disturbing infusion. (In one example, the reservoir is simply the same hollow-tube stylet **110,** which is long enough such that it holds enough fluid to fill the lumen of the infusion catheter **140** when the hollow-tube stylet **110** is withdrawn from the infusion catheter **140).** The hollow-tube stylet **110** is made of a material that is stiffer than that of the flexible catheter **140** to provide the catheter/stylet system **100** with sufficient torsional stability to allow tunneling and guiding through brain or other tissue toward a desired target.

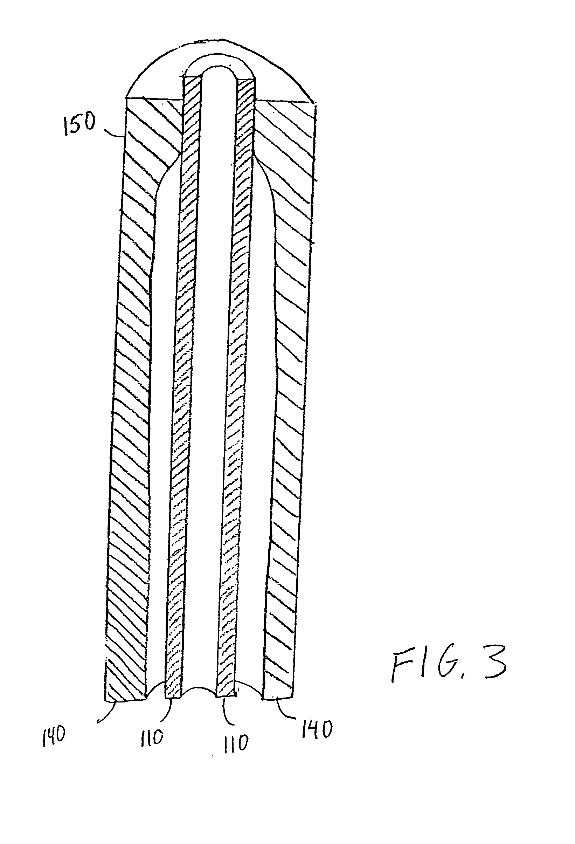

In one example, at least a proximal end **150** of the catheter **140** is optionally clamped (e.g., using encircling clamp **165)** or otherwise provides an inner diameter **170** that closes snugly around the outer diameter **175** of the hollow-tube stylet **110,** such as during removal of the hollow-tube stylet **110** from the catheter **140.** In another example, the inner diameter **170** of the entire catheter **140** is sized to snugly seal at least the proximal end **150** of the catheter **140** to the outer diameter **175** of the hollow-tube stylet **110.** In a further example, more distal portions (relative to the sealing proximal end) of the inner diameter of the catheter **140** are more loosely sized than the outer diameter of the hollow-tube stylet **110,** as illustrated in Figure **3****.** This allows easier sliding of the hollow-tube stylet **110** into and out of the catheter **140.** Figure 1 also illustrates an optional plug **180.** A portion of the plug **180** is sized and shaped to fit within the hollow stylet **110** to temporarily seal its proximal end to retain fluid within the hollow stylet **110.** The plug **180** is removed before the hollow stylet **110** is withdrawn from the catheter **140,** to permit the retained fluid to be released into the interior of the catheter. Alternatively, the proximal end of the hollow stylet **110** can be temporarily sealed using a cap or clamp, (or even a gloved finger, if desired). Moreover, if pinching or clamping is used, the sealing need not take place at the proximal end of the hollow stylet, but may be performed at a more intermediate portion of the hollow stylet **110.**

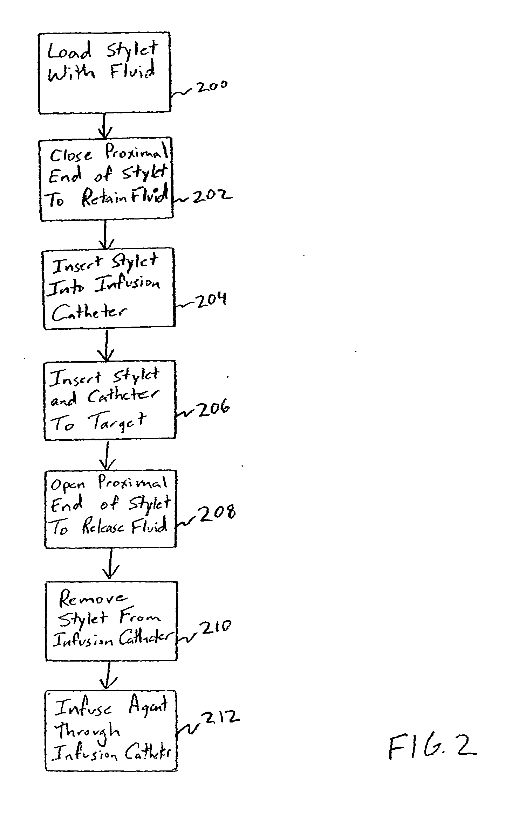

Figure 2 is a flow chart illustrating generally, by way of example, but not by way of limitation, one method of using the system **100.** In this example, at **200,** the hollow-tube stylet **110** is filled with fluid. At **202,** a proximal end of the hollow-tube stylet **110** is temporarily closed to retain the fluid within the hollow-tube stylet **110.** At **204,** the hollow-tube stylet **110** is then inserted into the infusion catheter **140.** (Alternatively, the hollow-tube stylet **110** is loaded with fluid, and then its proximal end closed, after it has been inserted into the infusion catheter **140).** At **206,** the hollow-tube stylet **110** and the catheter **140** are inserted together to the target location within the patient's brain or other tissue. (Alternatively, the stylet is inserted first, then the catheter is inserted over the guidewire stylet). The insertion of the stylet **110** and the catheter **140** may utilize an orientable and fixable trajectory guide, in conjunction with an image-guided surgical (IGS) workstation, to aim the stylet **110** and the catheter **140** toward a desired target. At **208,** the proximal end of the hollow-tube stylet **110** is opened to allow release of the fluid from therewithin. At **210,** the hollow-tube stylet **110** is withdrawn from within the infusion catheter **140.** The resulting vacuum draws the fluid out of the hollow-tube stylet **110** and into the infusion catheter **140,** thereby preventing or avoiding air from entering and remaining within the catheter **140** and disturbing the subsequent infusion. If desired, the catheter is then secured. At **212,** an agent is infused through the catheter 140 to the target location in the tissue.

In a further embodiment, the tunneling progress of the hollow-tube stylet **110** during surgery is tracked with one or more locators, such as a locator that is remotely detectable using a positioning system coupled to an image-guided surgical (IGS) workstation. In one such example, at least one remotely-detectable locator is attached to at least one of the hollow-tube stylet **110** and the catheter **140.** As the hollow-tube stylet **110** and the catheter **140** are tunneled through tissue toward a desired target, the locator's progress is tracked and displayed on a monitor of the IGS workstation.

It is to be understood that the above description is intended to be illustrative, and not restrictive. For example, the above-described embodiments may be used in combination with each other. Many other embodiments will be apparent to those of skill in the art upon reviewing the above description. The scope of the invention should, therefore, be determined with reference to the appended claims. . In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. A system comprising:
A flexible tubular infusion catheter (140) and a hollow tube (110), including a proximal end (120) and a distal end and a lumen extending therebetween, wherein the hollow tube (110) is shaped and sized to permit insertion into a lumen of the catheter (140), wherein the hollow tube (110) is stiffer than the infusion catheter (140) such that the hollow tube (110) acts as a stylet for guiding the catheter (140) through tissue to a target location, **characterized in that** the lumen of the hollow tube (110) is filled with a fluid, and wherein the proximal end (120) of the hollow tube (110) is configured to be close to retain the fluid within the lumen of the hollow tube (110).

2. The system of claim 1, further including a fluid reservoir (160) that is coupled to the proximal end (120) of the hollow tube (110).

3. The system of claim 3, wherein the hollow tube (110) and the fluid reservoir (160) are sized to hold enough fluid to fill the lumen of the infusion catheter (140) after withdrawal of the hollow tube (110) from the lumen of the infusion catheter (140).

4. The system of claim 1, further including a flexible tubular infusion catheter (140) including a proximal end (150) and a distal end and a lumen extending therebetween, the lumen of the infusion catheter (140) sized and shaped to permit insertion of the hollow tube (110) therein.

5. The system of claim 5, in which the proximal end of the infusion catheter (140) sealingly engages around the hollow tube (110) when a portion of the hollow tube (110) is located with the lumen of the infusion catheter (140).

6. The system of claim 6, in which the proximal end of the tubular catheter (140) includes a clamp (165) that closes around the hollow tube (110).

7. The system of claim 6, in which the lumen of the catheter (140) includes a diameter (170) having at least two different values at different locations along the lumen of the catheter (140).

8. The system of claim 1, in which the hollow tube (110) includes a remotely detectable locator.

9. The system of claim 9, further including a positioning system that permits location of the locator.

10. The system of claim 10, further including an image-guided surgical workstation coupled to the positioning system.

11. The system of claim 1, further including means for temporarily sealing the proximal end of the hollow tube (110) to retain fluid within the hollow tube (110).

## Patentansprüche

1. System umfassend:
einen flexiblen röhrenförmigen Infusionskatheter (140) und eine hohle Röhre (110), mit einem proximalen Ende (120), einem distalen Ende und einem sich dazwischen erstreckenden Hohlraum, wobei die hohle Röhre (110) so geformt und bemessen ist, dass ein Einbringen in einen Hohlraum des Katheters (140) ermöglicht wird, wobei die hohle Röhre (110) steifer ist als der Infusionskatheter (140), so dass die hohle Röhre (110) als ein Stilett fungiert, um den Katheter (140) durch Gewebe zu einem Zielort zu führen, **dadurch gekennzeichnet, dass** der Hohlraum der hohlen Röhre (110) mit einem Fluid gefüllt ist und das proximale Ende (120) der hohlen Röhre (110) verschließbar ausgestaltet ist, um das Fluid innerhalb des Hohlraums der hohlen Röhre (110) zurückzuhalten.

2. System gemäß Anspruch 1, mit ferner einem Fluidreservoir (160), welches mit dem proximalen Ende (120) der hohlen Röhre (110) gekoppelt ist.

3. System gemäß Anspruch 2, wobei die hohle Röhre (110) und das Fluidreservoir (160) so bemessen sind, dass sie genug Fluid beinhalten, um nach dem Zurückziehen der hohlen Röhre (110) aus dem Hohlraum des Infusionskatheters (140) den Hohlraum des Infusionskatheters (140) zu füllen.

4. System gemäß Anspruch 1, mit ferner einem flexiblen röhrenförmigen Infusionskatheter (140) mit einem proximalen Ende (150), einem distalen Ende und einem sich dazwischen erstreckenden Hohlraum, wobei der Hohlraum des Infusionskatheters (140) so bemessen und geformt ist, um das Einbringen der hohlen Röhre (110) in den Hohlraum ermöglicht wird.

5. System gemäß Anspruch 4, wobei das proximale Ende des Infusionskatheters (140) um die hohle Röhre (110) herum dichtend angreift, wenn ein Teil der hohlen Röhre (110) mit dem Hohlraum des Infusionskatheters (140) angeordnet ist.

6. System gemäß Anspruch 5, bei dem das proximale Ende des röhrenförmigen Katheters (140) eine Klammer (165) umfasst, die um die hohle Röhre (110) herum schließt.

7. System gemäß Anspruch 5, bei dem der Hohlraum des Katheters (140) einen Druchmesser (170) mit zumindest zwei verschiedenen Werten an verschiedenen Orten entlang des Hohlraums des Katheters (140) aufweist.

8. System gemäß Anspruch 1, bei dem die hohle Röhre (110) einen ferndetektierbaren Positionsgeber aufweist.

9. System gemäß Anspruch 8, mit ferner einem Positionierungssystem, welches die Lokalisierung des Positionsgebers erlaubt.

10. System gemäß Anspruch 9, mit ferner einer bildgeführten chirurgischen Arbeitsstation, die an das Positionierungssystem gekoppelt ist.

11. System gemäß Anspruch 1, mit ferner Mitteln zum vorübergehenden Abdichten des proximalen Endes der hohlen Röhre (110), um ein Fluid innerhalb der hohlen Röhre (110) zurückzuhalten.

## Revendications

1. Système, comprenant :
un cathéter d'injection tubulaire souple (140) et un tube creux (110), comprenant une extrémité proximale (120) et une extrémité distale et un conduit s'étendant entre celles-ci, le tube creux (110) étant conformé et dimensionné de façon à permettre l'insertion dans un conduit du cathéter (140), le tube creux (110) étant plus rigide que le cathéter d'injection (140), de telle sorte que le tube creux (110) joue le rôle de stylet pour guider le cathéter (140) à travers un tissu vers un emplacement cible, **caractérisé en ce que** le conduit du tube creux (110) est rempli d'un fluide, et dans lequel l'extrémité proximale (120) du tube creux (110) est configurée de façon à être fermée afin de maintenir le fluide à l'intérieur du conduit du tube creux (110).

2. Système selon la revendication 1, comprenant de plus un réservoir de fluide (160) qui est couplé à l'extrémité proximale (120) du tube creux (110).

3. Système selon la revendication 3, dans lequel le tube creux (110) et le réservoir de fluide (160) sont dimensionnés de façon à contenir suffisamment de fluide pour remplir le conduit du cathéter d'injection (140) après le retrait du tube creux (110) à partir du conduit du cathéter d'injection (140).

4. Système selon la revendication 1, comprenant de plus un cathéter d'injection tubulaire souple (140) comprenant une extrémité proximale (150) et une extrémité distale et un conduit s'étendant entre celles-ci, le conduit du cathéter d'injection (140) étant dimensionné et conformé de façon à permettre l'insertion du tube creux (110) à l'intérieur de celui-ci.

5. Système selon la revendication 5, dans lequel l'extrémité proximale du cathéter d'injection (140) vient en prise de façon étanche autour du tube creux (110) lorsqu'une partie du tube creux (110) est positionnée avec le conduit du cathéter d'injection (140).

6. Système selon la revendication 6, dans lequel l'extrémité proximale du cathéter tubulaire (140) comprend une pince (165) qui se ferme autour du tube creux (110).

7. Système selon la revendication 6, dans lequel le conduit du cathéter (140) comprend un diamètre (170) ayant au moins deux valeurs différentes en des emplacements différents le long du conduit du cathéter (140).

8. Système selon la revendication 1, dans lequel le tube creux (110) comprend un dispositif de localisation détectable à distance.

9. Système selon la revendication 9, comprenant de plus un système de positionnement qui permet la localisation du dispositif de localisation.

10. Système selon la revendication 10, comprenant de plus une station de travail chirurgicale à guidage par image couplée au système de positionnement.

11. Système selon la revendication 1, comprenant de plus des moyens pour sceller étanchement de façon temporaire l'extrémité proximale du tube creux (110) de façon à maintenir un fluide à l'intérieur du tube creux (110).
